# EUROPÄISCHE PATENTSCHRIFT

(11) **EP 2 526 082 B1**
(45) Veröffentlichungstag und Bekanntmachung des Hinweises auf die Patenterteilung: **25.12.2013**
(21) Anmeldenummer: 11700649.4
(22) Anmeldetag: 14.01.2011
(51) Int. Cl.: C07C 69/15, C07C 67/055, C07C 67/54

(54) **VERFAHREN ZUR HERSTELLUNG VON VINYLACETAT**
METHOD FOR PRODUCING VINYL ACETATE
PROCÉDÉ DE PRODUCTION D'ACÉTATE DE VINYLE

(30) Priorität: 21.01.2010 DE 102010001097
(43) Veröffentlichungstag der Anmeldung: 28.11.2012
(73) Patentinhaber: Wacker Chemie AG, 81737 München (DE)
(72) Erfinder: GUENALTAY, Mehmet, 84547 Emmerting (DE); DAFINGER, Willibald, 94133 Röhrnbach (DE); HOLL, Peter, 84547 Emmerting (DE)
(74) Vertreter: Ege, Markus
(86) Internationale Anmeldenummer: PCT/EP2011/050452
(87) Internationale Veröffentlichungsnummer: WO 2011/089070

(56) Entgegenhaltungen:
- DE-A1-102006 038 689

## Beschreibung

Die Erfindung betrifft Verfahren zur Herstellung von Vinylacetat in einem heterogen katalysierten, kontinuierlichen Gasphasenprozess durch Umsetzung von Ethylen mit Essigsäure und Sauerstoff, wobei die Aufarbeitung des dabei erhaltenen ProduktStromes effizienter ausgestaltet wird.

Der Herstellung von Vinylacetat durch Umsetzung von Ethylen mit Essigsäure und Sauerstoff oder sauerstoffhaltigen Gasen an einem Festbettkatalysator in der Gasphase ist seit langem bekannt. Die Edukte werden in einer exothermen Reaktion im Allgemeinen bei einem Druck von 1 bis 30 bar und einer Temperatur von 130°C bis 200°C in einem Festbett-Röhrenreaktor oder Wirbelschichtreaktor entsprechend der folgenden Bruttoformel zu Vinylacetat umgesetzt:

C₂H₄ + CH₃COOH + ½ O₂ → CH₃COOCH=CH₂ +H₂O.

Der aus dem Reaktor austretende Produktgasstrom enthält neben Vinylacetat im Wesentlichen nicht umgesetzte Edukte, Wasser sowie Inerten und Nebenprodukte, wie Kohlendioxid, Acetaldehyd, Methylacetat und Ethylacetat. Inerten sind im Wesentlichen Stickstoff, Argon, Methan und Ethan und werden als Verunreinigungen der Edukte in den Prozess eingebracht.

Zur Isolierung von reinem Vinylacetat aus dem Produktgasstrom sind eine Vielzahl von Verfahrensvarianten vorgeschlagen worden. In der DE-A 1282014 und DE-A 1668063 wird empfohlen, den Produktgasstrom in eine erste Destillationskolonne einzubringen, aus der über Kopf Wasser und Leichtsieder abdestilliert werden und der Sumpf in eine zweite Destillationskolonne überführt wird, aus der über Kopf reines Vinylacetat, abdestilliert wird. Zur Abtrennung von Methylacetat und Ethylacetat aus Gemischen mit Vinylacetat empfiehlt die DE-A 1618240 mit Wasser betriebene Extraktionsverfahren. Die US 3692636, US 4934519 und US 6228226 beschreiben Azeotropdestillationen von Vinylacetat, Wasser, Ethylacetat und Essigsäure enthaltenden Gemischen.

Im Verfahren der DE-A 1768412 werden aus dem Produktgasstrom kondensierbare Bestandteile, wie Vinylacetat, Essigsäure und Wasser, auskondensiert und als Kondensat in eine erste Destillationskolonne (Azeotropkolonne) eingebracht, aus der Vinylacetat und Wasser über Kopf abdestilliert und anschließend einer Phasentrennung unterzogen werden. Die Vinylacetat enthaltende Phase wird in eine zweite Destillationskolonne (Entwässerungskolonne) eingeleitet, deren Sumpf in eine dritte Destillationskolonne (Reinvinylacetatkolonne) überführt wird, aus der über Kopf reines Vinylacetat gewonnen wird. Zusätzlich zu den vorgenannten Verfahrensschritten wird in den Verfahren der DE-A 2945913 und der DE-A 2943985 die bei der Kondensation des Produktgasstromes zurückbleibende Gasphase mit Essigsäure (Kreisgaswäscher) gewaschen; die dabei erhaltene Waschlösung wird in die Azeotropkolonne eingebracht.

Zur energiesparenden Abtrennung von Wasser empfiehlt die DE-A 2610624, den Produktgasstrom in eine Vorentwässerungskolonne zu leiten, aus der über Kopf Wasser und Vinylacetat abdestilliert werden, die nach Kondensation und Phasentrennung separiert werden. Die so erhaltene Vinylacetat enthaltende Phase wird in die Vorentwässerungskolonne zurückgeführt oder gemeinsam mit dem Sumpf der Vorentwässerungskolonne in eine Entwässerungskolonne gegeben, deren Sumpf zur Gewinnung von reinem Vinylacetat weiteren destillativen Reinigungsschritten unterzogen wird. In den Verfahren der EP-A 1760065, DE-A 102006038689, DE 3934614, DE 3422575 und der US 4818347 wird die im vorigen beschriebene Vorentwässerungskolonne mit einem Essigsäure betriebenen Kreisgaswäscher, einer Azeotropkolonne und einer Entwässerungskolonne sowie einer Reinvinylacetatkolonne kombiniert, wobei aus der letzteren über Kopf das reine Vinylacetat gewonnen wird.

Vor diesem Hintergrund bestand weiterhin die Aufgabe, die Verfahren zur Isolierung von Vinylacetat aus dem Produktgasstrom effizienter auszugestalten, insbesondere hinsichtlich des Einsatzes von Energie oder Anlagenbauteilen.

Diese Aufgabe wurde überraschenderweise im Wesentlichen dadurch gelöst, dass der Produktgasstrom mittels je einer Vorentwässerungskolonne, einem mit Essigsäure betriebenen Kreisgaswäscher, einer Azeotropkolonne und einer Entwässerungskolonne gereinigt wurde, wobei das reine Vinylacetat aus dem Seitenabzug der Entwässerungskolonne gewonnen wurde. Im Gegensatz zum bisher bekannten Stand der Technik ist nach dem erfindungsgemäßen Verfahren vorteilhafterweise keine zusätzliche Reinvinylacetatkolonne erforderlich, aus der das reine Vinylacetat herkömmlicherweise über Kopf abdestilliert wird.

Gegenstand der Erfindung ist.ein Verfahren zur Herstellung von Vinylacetat in einem heterogen katalysierten, kontinuierlichen Gasphasenprozess durch Umsetzung von Ethylen mit Essigsäure und Sauerstoff in einem Reaktor, und Auftrennung des Produktgasstromes enthaltend im Wesentlichen Ethylen, Vinylacetat, Essigsäure, Wasser, Kohlendioxid und Inertgase, indem man
a) den Produktgasstrom in eine erste Destillationskolonne (Vorentwässerungskolonne) einleitet,
b) das am Kopf der ersten Destillationskolonne austretende Gasgemisch auf -20 bis +50°C abkühlt, wobei das anfallende Kondensat eine Wasserphase und eine organische Phase bildet,
c) die in Schritt b) gebildete Wasserphase abzieht,
d) die in Schritt b) gebildete organische Phase ganz oder teilweise in einen Sammelbehälter gibt oder als Rücklauf auf den Kopf der ersten Destillationskolonne in Schritt a) gibt,
e) das in Schritt b) nicht kondensierte Gas in einer mit Essigsäure betriebenen Waschkolonne (Kreisgaswäscher) wäscht und am Sumpf der Waschkolonne eine Vinylacetat-haltige Essigsäurelösung abgezogen wird,
f) die Vinylacetat-haltige Essigsäurelösung aus Schritt e) in eine zweite Destillationskolonne (Azeotropkolonne) einleitet,
g) das Sumpfprodukt aus Schritt a) ebenfalls in die zweite Destillationskolonne f) einbringt,
h) den Kopfdampf der zweiten Destillationskolonne abkühlt, wobei sich eine Wasserphase und eine organische Phase bilden,
i) die in Schritt h) gebildete wässerige Phase abzieht,
j) gegebenenfalls einen Teil der in Schritt h) gebildeten organischen Phase als Rücklauf auf den Kopf der zweiten Destillationskolonne f) zurückführt,
k) den restlichen Teil der in Schritt h) gebildeten organischen Phase in den in Schritt d) genannten Sammelbehälter gibt,
l) die Flüssigkeit aus dem in den Schritten k) und d) genannten Sammelbehälter in eine dritte Destillationskolonne (Entwässerungskolonne) einleitet, dadurch gekennzeichnet, dass m) Vinylacetat als Seitenabzug aus der dritten Destillationskolonne aus Schritt 1) abgetrennt wird.

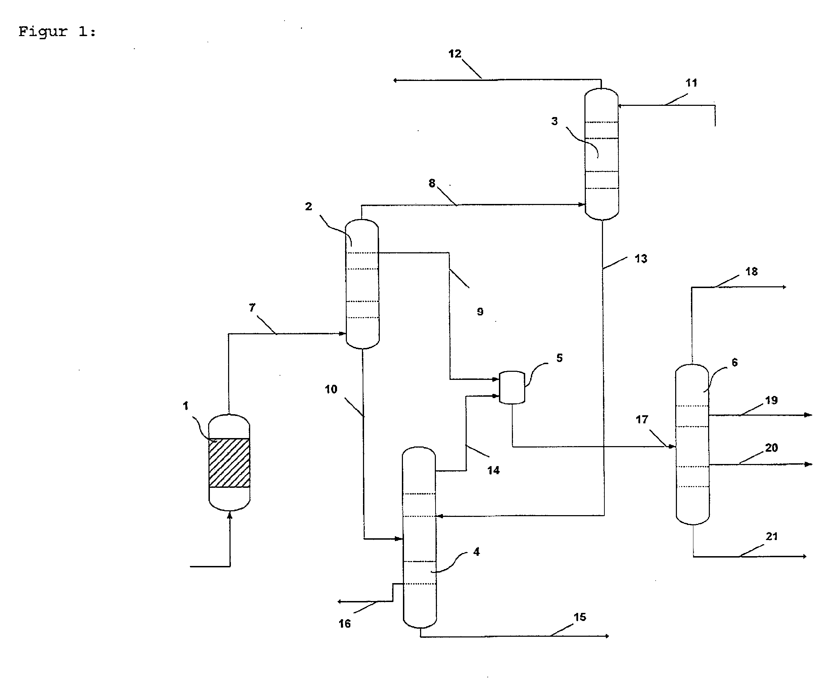

In Figur 1 ist ein vereinfachtes Schema für das erfindungsgemäße Verfahren angegeben.

Bei der kontinuierlichen Herstellung von Vinylacetat wird vorzugsweise in Röhrenreaktoren gearbeitet, welche mit einem Festbettkatalysator beschickt sind. Diese Katalysatoren sind im Allgemeinen mit Edelmetall(salz)en und Promotoren dotierte Trägerkatalysatoren, beispielsweise mit Palladium und mit Gold und Kalium-Salzen dotierte Bentonit-Kugeln. Der Reaktor (1) wird mit Ethylen, Sauerstoff und Essigsäure beschickt und die Reaktion bei einem Druck von vorzugsweise 8 bis 12 bar abs. (Kreisgasdruck) und einer Temperatur von vorzugsweise 130 bis 200°C durchgeführt.

Der aus dem Reaktor (1) austretende Produktgasstrom (7) enthält im Wesentlichen Vinylacetat, Ethylen, Essigsäure, Wasser, Sauerstoff und Nebenprodukte, wie Kohlendioxid und Ethylacetat, sowie Inerten, wie Stickstoff, Argon, Methan und Ethan. Der Produktgasstrom (7) wird vorzugsweise unmittelbar, gegebenenfalls aber nach Temperierung auf 115 bis 130°C, in die erste Destillationskolonne (Vorentwässerungskolonne) (2) eingeleitet (Schritt a)). Die Vorentwässerungskolonne (2) wird vorzugsweise unter Kreisgasdruck betrieben. Dies ist aus energetischen Gründen vorteilhaft, da der Produktgasstrom (7) erhebliche Mengen an Ethylen enthält, das auf diese Weise mit geringstmöglichem Dekomprimierungs- bzw. Komprimierungsaufwand in den Reaktor (1) zurückgeführt werden kann, Die Betriebstemperatur und der Rücklauf der Vorentwässerungskolonne (2) sind vorzugsweise so zu wählen, dass möglichst der gesamte Ethylacetat-Anteil des Produktgasstromes (7) im Sumpf der Vorentwässerungskolonne (2) angesammelt wird. Die Betriebstemperatur im Sumpf der Vorentwässerungskolonne (2) beträgt vorzugsweise von 100 bis 120 °C.

Die in Schritt b) gebildete organische Phase (9) enthält üblicherweise 90 bis 99, insbesondere 95 bis 98 Gew.-% Vinylacetat, ≤ 3 Gew.-% Acetaldehyd, 0,5 bis 8 Gew. -%, insbesondere 1 bis 2 Gew.-% Wasser, ≤ 250 ppm Ethylacetat sowie ≤ 250 ppm Methylacetat, wobei sich die Angaben in Gew.-% auf das Gesamtgewicht der organischen Phase aus Schritt b) (9) beziehen. Die organische Phase aus Schritt b) (9) enthält also im Wesentlichen keine Essigsäure, d.h. vorzugsweise ≤ 15 ppm, besonders bevorzugt ≤ 10 ppm, und am meisten bevorzugt ≤ 5 ppm Essigsäure.

Das in Schritt b) nicht kondensierte Gas (8) besteht im Wesentlichen aus Ethylen und CO₂ und geringen Mengen an Vinylacetat, wird in dem mit Essigsäure (11) betriebenen Kreisgaswäscher (3) von kondensierbaren Anteilen befreit (Schritt e)). Das Betreiben von Kreisgaswäschern an sich ist dem Fachmann bekannt. Der am Kopf des Kreisgaswäschers (3) entnommene Kreisgasstrom (12) wird im Allgemeinen ganz oder teilweise, gegebenenfalls nach Reinigung oder Komprimierungsschritten als Kreisgas in den Reaktor (1) zurückgeführt. Am Sumpf des Kreisgaswäschers (3) sammelt sich eine Vinylacetat-haltige Essigsäurelösung an, aus der ein Strom teilweise oder vorzugsweise ganz (13) in eine zweite Destillationskolonne (Azeotropkolonne) (4) eingebracht wird (Schritt f)).

Im Bereich des Kopfes der Azeotropkolonne (4) beträgt der Druck üblicherweise 1,1 bis 1,5 bar abs. und die Temperatur 50 bis 90°C. Die in Schritt h) gebildete organische Phase (14) enthält üblicherweise 90 bis 99, insbesondere 95 bis 98 Gew.-% Vinylacetat, ≤ 3 Gew.-% Acetaldehyd, 0, 5 bis 8 Gew.-%, insbesondere 1 bis 2 Gew.-% Wasser, ≤ 250 ppm Ethylacetat sowie ≤ 250 ppm Methylacetat, wobei sich die Angaben in Gew.-% auf das Gesamtgewicht der organischen Phase aus Schritt h) (14) beziehen. Die organische Phase aus Schritt h) (14) enthält also im Allgemeinen im Wesentlichen keine Essigsäure, d.h. vorzugsweise ≤ 15 ppm, besonders bevorzugt ≤ 10 ppm und am meisten bevorzugt ≤ 5 ppm Essigsäure.

Üblicherweise wird aus einer Anreicherungszone oberhalb vom Sumpf der Azeotropkolonne (4) ein ethyl- und/oder methylacetathaltiger Seitenstrom (16) abgezogen. Alternativ kann Ethylacetat und/oder Methylacetat auch aus dem Sumpf (15) der Azeotropkolonne (4) entnommen und in einer separaten Destillationskolonne abdestilliert werden. Der Sumpf (15) der Azeotropkolonne (4) enthält im Wesentlichen Essigsäure und wird vorzugsweise ganz oder teilweise in den Kreisgaswäscher (3) in Schritt e) als Strom (11) oder, gegebenenfalls nach einer weiteren Reinigung, in den Reaktor (1) zurückgeführt.

In den im Sammelbehälter (5) zusammengefassten, in den Schritten d) und k) gesammelten organischen Phasen (9) und (14) sind vorzugsweise ≤ 25 ppm, besonders bevorzugt ≤ 10 ppm und am meisten bevorzugt ≤ 5 ppm Essigsäure enthalten.

Im Bereich des Kopfes der Entwässerungskolonne (6) beträgt der Druck üblicherweise 1,1 bis 2 bar abs. und die Temperatur 50 bis 90°C, inbsbesondere 70 bis 80°C.

Wesentlich für das erfindungsgemäße Verfahren ist, dass die Entnahme des gereinigten Vinylacetats im Abtriebsteil (20) der Entwässerungskolonne (6) erfolgt, bevorzugt zwischen dem fünften und zwanzigsten Boden oberhalb des Sumpfes der Entwässerungskolonne (6). Das Vinylacetat kann hierbei in Form eines Gases oder in flüssiger Form aus der Entwässerungskolonne (6) entnommen werden - je nach dem, an welchem Boden der Entwässerungskolonne der Seitenabzug (20) erfolgt. In Form eines Gases wird Vinylacetat vorzugsweise dem fünften bis fünfzehnten Boden der Entwässerungskolonne (6) entnommen. Für den Fall, dass bei der Destillation ein Inhibitor zugegen ist, erfolgt der Seitenabzug von Vinylacetat (20) aus der Entwässerungskolonne (6) vorzugsweise in Form eines Gases. Inhibitoren werden üblicherweise zur Vermeidung von Polymerisation eingesetzt. Gängige Inhibitoren sind z.B. Chinone.

Das in Schritt m) erhaltene Produkt enthält vorzugsweise ≥ 99 Gew.-%, besonders bevorzugt ≥ 99,5 Gew.-% und am meisten bevorzugt ≥ 99,95 Gew.-% Vinylacetat, jeweils bezogen auf die Gesamtmasse des Produkts. Des Weiteren kann das in Schritt m) erhaltene Produkt als Nebenkomponenten beispielsweise ≤ 100 ppm Wasser, ≤ 50 ppm Essigsäure, ≤ 300 ppm Ethylacetat oder ≤ 50 ppm Aldehyde, wie Acetaldehyd, enthalten. Die Hazen-Zahl ist ≤ 10 (Bestimmung gemäß DIN 55945 mit einem Spektralphotometer). Die Hazen-Zahl ist ein gängiger Kennwert für die Farbe transparenter Stoffe.

Im Zuge der Destillation in der Entwässerungskolonne (6) kann durch Hydrolyse von Vinylacetat in geringem Umfang Essigsäure gebildet werden. Durch Entnahme von Vinylacetat vom Seitenabzug (20) der Entwässerungskolonne (6) kann aber gewährleistet werden, dass das isolierte Vinylacetat im Wesentlichen keine Essigsäure enthält. Das erfindungsgemäß hergestellte Vinylacetat enthält vorzugsweise ≤ 50 ppm, besonders bevorzugt ≤ 25 ppm und am meisten bevorzugt ≤ 5 ppm Essigsäure.

Das nach dem erfindungsgemäßen Verfahren hergestellte Vinylacetat liegt in der für technische Anwendungen geforderten Reinheit vor, und dies überraschenderweise, obwohl nicht wie im bisherigen Stand der Technik üblich nach der Entwässerungskolonne eine zusätzliche Reinvinylacetatkolonne zum Einsatz kommt, aus der im Stand der Technik Vinylacetat über Kopf abdestilliert wird. Deswegen kann nach dem erfindungsgemäßen Verfahren im Vergleich zum Stand der Technik die Destillationsanlage für die Reinvinylacetatkolonne und die damit verbunden Aufwendungen für deren Instandhaltung und Betrieb, wie Energie und Wasserdampf, gespart werden. Dies führt zu einer erheblichen Reduzierung der Investitions- und Betriebskosten. Das nachfolgende Beispiel dient der weiteren Erläuterung der Erfindung, ohne die Erfindung in irgendeiner Weise einzuschränken:

In einer Anlage gemäß Figur 1 wurde entsprechend der oben beschriebenen Vorgehensweise und Bedingungen im Sammerbehälter (5) eine organische Phase mit folgender Zusammensetzung gesammelt: 96 Gew.-Teile Vinylacetat, 2,4 Gew.-Teile Acetaldehyd, 1,6 Gew.-Teile Wasser und jeweils 250 bis 300 ppm Ethyl- und Methylacetat. Diese organische Phase (17) wurde in die Entwässerungskolonne (6) eingebracht. Die Leichtsieder und das Wasser wurden im Verstärkerteil (18) bzw. (19) abgetrennt. Oberhalb des Sumpfes, aber unterhalb von (18) und (19) wurde ein Seitenstrom (20) abgezogen. Der Seitenstrom (20) bestand zu 99,998 Gew.-% aus Vinylacetat und enthielt Spuren von Ethylacetat (200 ppm), Methylacetat (150 ppm) und Essigsäure (30 ppm).
Der Gesamtmassenstrom des Sumpfabzugs (21) betrug 0,5% des Zulaufmassenstroms (17); der Sumpfabzug wurde der Azeotropdestillation zurückgeführt.

## Patentansprüche

1. Verfahren zur Herstellung von Vinylacetat in einem heterogen katalysierten, kontinuierlichen Gasphasenprozess durch Umsetzung von Ethylen mit Essigsäure und Sauerstoff in einem Reaktor, und Auftrennung des Produktgasstromes enthaltend im Wesentlichen Ethylen, Vinylacetat, Essigsäure, Wasser, Kohlendioxid und Inertgase, indem man
a) den Produktgasstrom in eine erste Destillationskolonne (Vorentwässerungskolonne) einleitet,
b) das am Kopf der ersten Destillationskolonne austretende Gasgemisch auf -20 bis +50°C abkühlt, wobei das anfallende Kondensat eine Wasserphase und eine organische Phase bildet,
c) die in Schritt b) gebildete Wasserphase abzieht,
d) die in Schritt b) gebildete organische Phase ganz oder teilweise in einen Sammelbehälter gibt oder als Rücklauf auf den Kopf der ersten Destillationskolonne in Schritt a) gibt,
e) das in Schritt b) nicht kondensierte Gas in einer mit Essigsäure betriebenen Waschkolonne (Kreisgaswäscher) wäscht und am Sumpf der Waschkolonne eine Vinylacetat-haltige Essigsäurelösung abgezogen wird,
f) die Vinylacetat-haltige Essigsäurelösung aus Schritt e) in eine zweite Destillationskolonne (Azeotropkolonne) einleitet,
g) das Sumpfprodukt aus Schritt a) ebenfalls in die zweite Destillationskolonne f) einbringt,
h) den Kopfdampf der zweiten Destillationskolonne abkühlt, wobei sich eine Wasserphase und eine organische Phase bilden,
i) die in Schritt h) gebildete wässerige Phase abzieht,
j) gegebenenfalls einen Teil der in Schritt h) gebildeten organischen Phase als Rücklauf auf den Kopf der zweiten Destillationskolonne f) zurückführt,
k) den restlichen Teil der in Schritt h) gebildeten organischen Phase in den in Schritt d) genannten Sammelbehälter gibt,
l) die Flüssigkeit aus dem in den Schritten k) und d) genannten Sammelbehälter in eine dritte Destillationskolonne (Entwässerungskolonne) einleitet, **dadurch gekennzeichnet, dass**
m) Vinylacetat als Seitenabzug aus der dritten Destillationskolonne aus Schritt l) abgetrennt wird.

2. Verfahren zur Herstellung von Vinylacetat nach Anspruch 1, **dadurch gekennzeichnet, dass** die in Schritt b) gebildete organische Phase 90 bis 99 Gew.-% vinylacetat, ≤ 2 Gew.-% Acetaldehyd, 0,5 bis 8 Gew.-% Wasser, ≤ 250 ppm Ethylacetat sowie ≤ 250 ppm Methylacetat enthält, wobei sich die Angaben in Gew.-% auf das Gesamtgewicht der organischen Phase aus Schritt b) beziehen.

3. Verfahren zur Herstellung von Vinylacetat nach Anspruch 1 oder 2, **dadurch gekennzeichnet, dass** die in Schritt h) gebildete organische Phase 90 bis 99 Gew.-% Vinylacetat, ≤ 2 Gew.-% Acetaldehyd, 0,5 bis 8 Gew.-% Wasser, ≤ 250 ppm Ethylacetat sowie ≤ 250 ppm Methylacetat enthält, wobei sich die Angaben in Gew.-% auf das Gesamtgewicht der organischen Phase aus Schritt h) beziehen.

4. Verfahren zur Herstellung von Vinylacetat nach Anspruch 1 bis 3, **dadurch gekennzeichnet, dass** die im Sammelbehälter aus den Schritten d) und k) gesammelten organischen Phasen ≤ 25 ppm Essigsäure enthalten.

5. Verfahren zur Herstellung von Vinylacetat nach Anspruch 1 bis 4, **dadurch gekennzeichnet, dass** das Vinylacetat in Schritt m) in Form eines Gases oder in flüssiger Form aus der dritten Destillationskolonne entnommen wird.

6. Verfahren zur Herstellung von Vinylacetat nach Anspruch 1 bis 5, **dadurch gekennzeichnet, dass** das in Schritt m) abgetrennte Produkt ≥ 99 Gew.-% Vinylacetat enthält, bezogen auf die Gesamtmasse des Produkts.

7. Verfahren zur Herstellung von Vinylacetat nach Anspruch 1 bis 6, **dadurch gekennzeichnet, dass** das in Schritt m) abgetrennte Produkt ≤ 100 ppm Wasser Essigsäure, ≤ 300 ppm Ethylacetat und ≤ 50 ppm Aldehyde enthält.

8. Verfahren zur Herstellung von Vinylacetat nach Anspruch 1 bis 7, **dadurch gekennzeichnet, dass** das in Schritt m) abgetrennte Produkt ≤ 50 ppm Essigsäure enthält.

## Claims

1. Process for preparing vinyl acetate in a heterogeneously catalyzed, continuous gas-phase process by reacting ethylene with acetic acid and oxygen in a reactor and fractionating the product gas stream containing essentially ethylene, vinyl acetate, acetic acid, water, carbon dioxide and inert gases, by
a) introducing the product gas stream into a first distillation column (preliminary dewatering column),
b) cooling the gas mixture exiting at the top of the first distillation column to from -20 to +50°C, with the condensate obtained forming an aqueous phase and an organic phase,
c) taking off the aqueous phase formed in step b),
d) introducing all or part of the organic phase formed in step b) into a collection vessel or feeding it as runback to the top of the first distillation column in step a),
e) scrubbing the gas which has not been condensed in step b) in a scrubbing column (recycle gas scrubber) operated using acetic acid and taking off an acetic acid solution containing vinyl acetate at the bottom of the scrubbing column,
f) introducing the acetic acid solution containing vinyl acetate from step e) into a second distillation column (azeotropic column),
g) introducing the bottom product from step a) likewise into the second distillation column f),
h) cooling the overhead vapour from the second distillation column to form an aqueous phase and an organic phase,
i) taking off the aqueous phase formed in step h),
j) optionally recirculating part of the organic phase formed in step h) as runback to the top of the second distillation column f),
k) introducing the remaining part of the organic phase formed in step h) into the collection vessel named in step d),
l) introducing the liquid from the collection vessel named in steps k) and d) into a third distillation column (dewatering column), **characterized in that**
m) vinyl acetate is separated off as side offtake stream from the third distillation column of step l).

2. Process for preparing vinyl acetate according to Claim 1, **characterized in that** the organic phase formed in step b) contains from 90 to 99% by weight of vinyl acetate, ≤ 2% by weight of acetaldehyde, from 0.5 to 8% by weight of water, ≤ 250 ppm of ethyl acetate and ≤ 250 ppm of methyl acetate, where the values in % by weight are based on the total weight of the organic phase from step b) .

3. Process for preparing vinyl acetate according to Claim 1 or 2, **characterized in that** the organic phase formed in step h) contains from 90 to 99% by weight of vinyl acetate, ≤ 2% by weight of acetaldehyde, from 0.5 to 8% by weight of water, ≤ 250 ppm of ethyl acetate and ≤ 250 ppm of methyl acetate, where the values in % by weight are based on the total weight of the organic phase from step h) .

4. Process for preparing vinyl acetate according to any of Claims 1 to 3, **characterized in that** the organic phases collected in the collection vessel from steps d) and k) contain ≤ 25 ppm of acetic acid.

5. Process for preparing vinyl acetate according to any of Claims 1 to 4, **characterized in that** the vinyl acetate is taken off in the form of a gas or in liquid form from the third distillation column in step m).

6. Process for preparing vinyl acetate according to any of Claims 1 to 5, **characterized in that** the product separated off in step m) contains ≥ 99% by weight of vinyl acetate, based on the total mass of the product.

7. Process for preparing vinyl acetate according to any of Claims 1 to 6, **characterized in that** the product separated off in step m) contains ≤ 100 ppm of water, acetic acid, ≤ 300 ppm of ethyl acetate and ≤ 50 ppm of aldehydes.

8. Process for preparing vinyl acetate according to any of Claims 1 to 7, **characterized in that** the product separated off in step m) contains ≤ 50 ppm of acetic acid.

## Revendications

1. Procédé pour la production d'acétate de vinyle dans un processus continu en phase gazeuse à catalyse hétérogène, par mise en réaction d'éthylène avec de l'acide acétique et de l'oxygène dans un réacteur, et séparation du courant de gaz produit contenant essentiellement de l'éthylène, de l'acétate de vinyle, de l'acide acétique, de l'eau, du dioxyde de carbone et des gaz inertes, par
a) introduction du courant de gaz produit dans une première colonne de distillation (colonne de pré-déshydratation),
b) refroidissement jusqu'à -20 à +50 °C du mélange de gaz sortant à la tête de la première colonne de distillation, le condensat produit formant une phase aqueuse et une phase organique,
c) décharge de la phase aqueuse formée dans l'étape b),
d) introduction de la phase organique formée dans l'étape b) en partie ou en totalité dans un récipient collecteur ou envoi de celle-ci en tant que courant de recyclage à la tête de la première colonne de distillation dans l'étape a),
e) lavage du gaz non condensé dans l'étape b), dans une colonne de lavage fonctionnant avec de l'acide acétique (laveur de gaz en circuit) et décharge au pied de la colonne de lavage d'une solution d'acide acétique contenant de l'acétate de vinyle,
f) introduction de la solution d'acide acétique contenant de l'acétate de vinyle, provenant de l'étape e), dans une deuxième colonne de distillation (colonne azéotropique),
g) introduction du produit de pied provenant de l'étape a) également dans la deuxième colonne de distillation f),
h) refroidissement de la vapeur à la tête de la deuxième colonne de distillation, avec formation d'une phase aqueuse et d'une phase organique,
i) décharge de la phase aqueuse formée dans l'étape h),
j) éventuellement recyclage d'une partie de la phase organique formée dans l'étape h), en tant que courant de recyclage, à la tête de la deuxième colonne de distillation f),
k) introduction de la partie restante de la phase organique, formée dans l'étape h), dans le récipient collecteur nommé dans l'étape d),
l) introduction du liquide provenant du récipient collecteur, nommé dans les étapes k) et d), dans une troisième colonne de distillation (colonne de déshydratation), **caractérisé en ce que**
m) on sépare l'acétate de vinyle en tant que courant de décharge latéral de la troisième colonne de distillation de l'étape 1).

2. Procédé pour la production d'acétate de vinyle selon la revendication 1, **caractérisé en ce que** la phase organique formée dans l'étape b) contient 90 à 99 % en poids d'acétate de vinyle, ≤ 2 % en poids d'acétaldéhyde, 0,5 à 8 % en poids d'eau, ≤ 250 ppm d'acétate d'éthyle ainsi que ≤ 250 ppm d'acétate de méthyle, les données en % en poids se rapportant au poids total de la phase organique provenant de l'étape b).

3. Procédé pour la production d'acétate de vinyle selon la revendication 1 ou 2, **caractérisé en ce que** la phase organique formée dans l'étape h) contient 90 à 99 % en poids d'acétate de vinyle, ≤ 2 % en poids d'acétaldéhyde, 0,5 à 8% en poids d'eau, ≤ 250 ppm, d'acétate d'éthyle ainsi que ≤ 250 ppm d'acétate de méthyle, les données en % en poids se rapportant au poids total de la phase organique provenant de l'étape h) .

4. Procédé pour la production d'acétate de vinyle selon les revendications 1 à 3, **caractérisé en ce que** les phases organiques recueillies dans le récipient collecteur en provenance des étapes d) et k) contiennent ≤ 25 ppm d'acide acétique.

5. Procédé pour la production d'acétate de vinyle selon les revendications 1 à 4, **caractérisé en ce que** dans l'étape m) l'acétate de vinyle est prélevé de la troisième colonne de distillation sous forme d'un gaz ou sous forme liquide.

6. Procédé pour la production d'acétate de vinyle selon les revendications 1 à 5, **caractérisé en ce que** le produit séparé dans l'étape m) contient ≥ 99 % en poids d'acétate de vinyle, par rapport à la masse totale du produit.

7. Procédé pour la production d'acétate de vinyle selon les revendications 1 à 6, **caractérisé en ce que** le produit séparé dans l'étape m) contient ≤ 100 ppm d'eau, de l'acide acétique, ≤ 300 ppm d'acétate d'éthyle et ≤ 50 ppm d'aldéhydes.

8. Procédé pour la production d'acétate de vinyle selon les revendications 1 à 7, **caractérisé en ce que** le produit séparé dans l'étape m) contient ≤ 50 ppm d'acide acétique.
